# EUROPEAN PATENT SPECIFICATION

(11) **EP 3 380 458 B1**
(45) Date of publication and mention of the grant of the patent: **24.07.2019**
(21) Application number: 16788700.9
(22) Date of filing: 02.11.2016
(51) Int. Cl.: C07D 301/12

(54) **PROCESS FOR THE EPOXIDATION OF PROPENE**
VERFAHREN ZUR EPOXIDIERUNG VON PROPEN
PROCEDE D'EPOXYDATION DE PROPENE

(30) Priority: 26.11.2015 EP 15196517
(43) Date of publication of application: 03.10.2018
(73) Proprietor: Evonik Degussa GmbH, 45128 Essen (DE); thyssenkrupp Industrial Solutions AG, 45143 Essen (DE)
(72) Inventor: HOFEN, Willi, 63517 Rodenbach (DE); HAAS, Thomas, 48161 Münster (DE); WÖLL, Wolfgang, 63477 Maintal (DE); KOLBE, Bärbel, 58452 Witten (DE)
(74) Representative: Thiele, Georg Friedrich
(86) International application number: PCT/EP2016/076319
(87) International publication number: WO 2017/089079

(56) References cited:
- WO-A1-01/57010
- WO-A1-2005/103024
- CN-A- 101 817 804

## Description

### Field of the invention

The present invention relates to a process for the continuous epoxidation of propene with hydrogen peroxide in the presence of an epoxidation catalyst, where non-reacted propene is recovered and recycled to the epoxidation reaction.

### Background of the invention

The epoxidation of propene with hydrogen peroxide in the presence of an epoxidation catalyst is usually carried out with a molar excess of propene relative to hydrogen peroxide in order to avoid hydrogen peroxide decomposition and to achieve high selectivities for propene oxide. The reaction is usually carried out at a pressure of more than 1.0 MPa to achieve high propene concentrations in the liquid phase reaction mixture. Epoxidation of propene with a heterogeneous titanium silicalite catalyst is known from EP 0 100 119 A1. Epoxidation of propene with a homogeneous manganese catalyst is known from WO 2011/063937. Epoxidation of propene with a homogeneous tungstophosphate catalyst is known from US 5,274,140.

For an efficient use of propene, non-reacted propene has to be recovered from the reaction mixture of the epoxidation reaction and recycled to the epoxidation reaction.

EP 0 719 768 A1 describes recovery of non-reacted propene by a distillation where essentially all propene oxide remains in the bottoms product.

WO 99/07690 suggests to substantially remove unreacted propene from an epoxidation reaction product by distillation such as a flash distillation.

WO 2008/118265 A1 teaches that two depropanization columns running at low and high pressures are required to effectively separate propane and propene from the epoxidation reaction mixture without significant loss of propene oxide. As an alternative, a combination of a flash separator and an extractive distillation using methanol and/or water as extractive distillation solvent is proposed.

WO2005/103024 describes recovery of non-reacted propene by distillation at low pressure, preferably in a single column, providing a propene rich vapor as overhead product, compressing this vapor in two or three compression stages and absorbing propene from the compressed vapor with a liquid absorbent.

WO 01/57010 describes recovery of non-reacted propene by a pressure release stage, recompressing the gas obtained in this stage to the pressure prevailing in the epoxidation reactor and returning the gas to the reaction. Further propene can be recovered in vapor form by a subsequent separation of the liquid obtained in the pressure release stage in a pre-evaporator, partial condensation of the overhead product obtained in the pre-evaporator and recompression of the uncondensed propene.

Recovery of non-reacted propene by distillation at a high pressure that allows for condensing propene with cooling water has the disadvantage that mixtures containing propene oxide, water and solvent are subjected to high temperatures for extended periods at the bottom of the distillation column, which causes loss of propene oxide due to hydrolysis and solvolysis. Recovery of non-reacted propene at a low pressure requires recompression of propene to the pressure used in the epoxidation reaction which is energy intensive. Reference is also made to CN 101817804.

### Summary of the invention

It has now been found that the energy consumption for propene recovery by pressure reduction of the epoxidation reaction mixture can be reduced considerably by carrying out the pressure reduction in a series of several pressure reduction steps and recompressing the vapors resulting from pressure reduction in these steps in a corresponding series of compression steps in reverse order, where after each compression step the compressed vapor is combined with the vapor resulting from the preceding pressure reduction step.

Subject of the invention is therefore a process for the epoxidation of propene, comprising
a) continuously reacting a propene feed with hydrogen peroxide in the presence of an epoxidation catalyst in a reaction step at a pressure of at least 1.9 MPa, using propene in excess to hydrogen peroxide, to provide a liquid reaction mixture comprising non-reacted propene;
b) subjecting the liquid reaction mixture to a series of at least two sequential pressure reduction steps, the series comprising a first pressure reduction step and a last pressure reduction step, each pressure reduction step reducing the pressure from an initial pressure to a final pressure and providing a vapor phase, comprising non-reacted propene, and a liquid phase at the final pressure, the final pressure of the last pressure reduction step being less than 0.3 MPa;
c) compressing for each pressure reduction step the vapor phase provided by said step in a compression step to provide a compressed vapor phase having at least the initial pressure of said pressure reduction step, combining for each pressure reduction step, except the last pressure reduction step, the vapor phase provided by said step with compressed vapor phase from the subsequent pressure reduction step before performing said compression step;
d) condensing the compressed vapor phase from the first pressure reduction step in a condensing step to recover non-reacted propene and recycling at least part of the recovered non-reacted propene to the reaction step; and
e) separating propene oxide from the liquid phase provided in the last pressure reduction step.

### Brief description of drawings

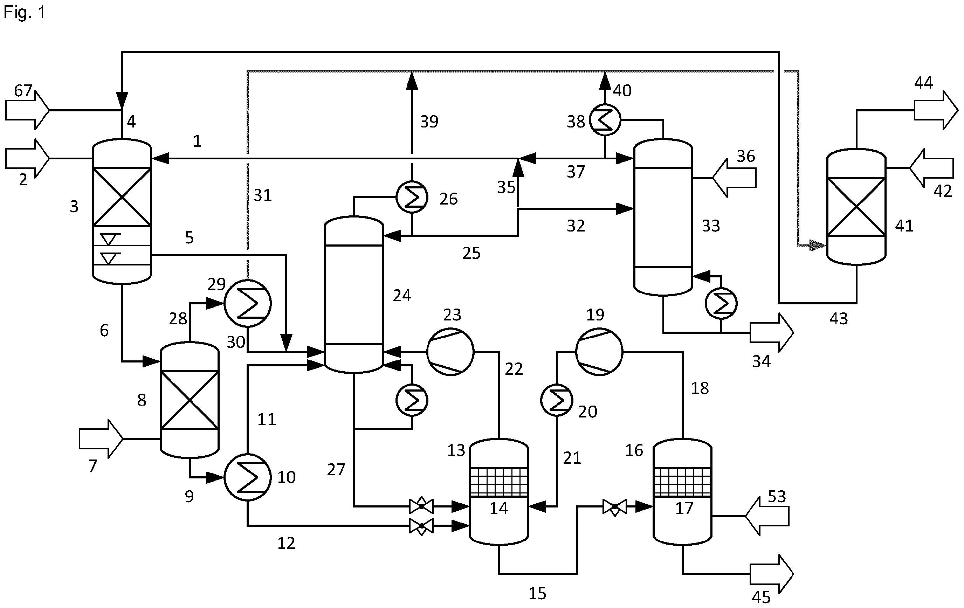

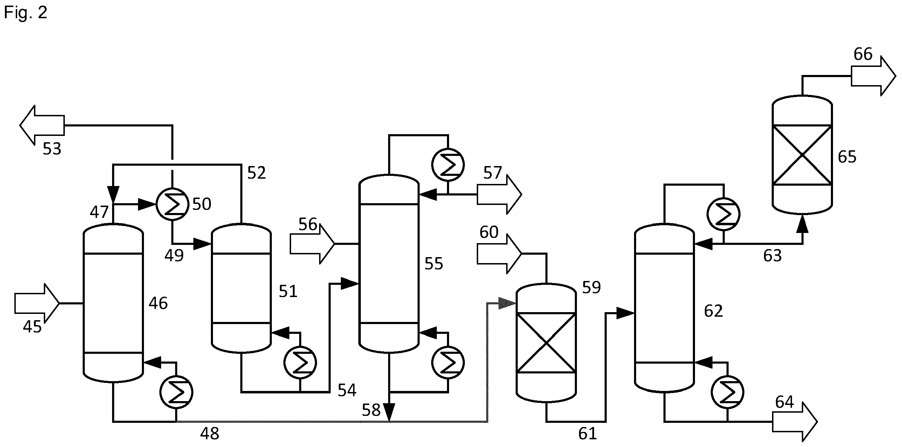

Figures 1 and 2 show a preferred embodiment of the process of the invention with two pressure reduction steps and a methanol solvent.

### Detailed description of the invention

All pressure values refer to absolute pressure in Megapascal (MPa).

In step a) of the process of the invention, a propene feed is continuously reacted in a reaction step with hydrogen peroxide in the presence of an epoxidation catalyst to provide a reaction mixture containing propene oxide and non-reacted propene. The reaction is carried out at a pressure of at least 1.9 MPa using propene in excess to hydrogen peroxide. Propene is preferably used at a molar ratio of propene to hydrogen peroxide of from 1.1:1 to 30:1, more preferably 2:1 to 10:1 and most preferably 3:1 to 5:1. Propene is preferably used in an excess sufficient to maintain an additional liquid phase rich in propene throughout step a). The pressure in step a) is preferably from 1.9 to 5.0 MPa, more preferably 2.1 to 3.6 MPa and most preferably 2.4 to 2.8 MPa. Using an excess of propene at a high pressure provides high reaction rate and hydrogen peroxide conversion and at the same time high selectivity for propene oxide.

The propene feed may contain propane, preferably with a molar ratio of propane to propene of from 0.001 to 0.15 and more preferably of from 0.08 to 0.12. Hydrogen peroxide can be used as an aqueous solution, preferably containing from 30 to 75 % by weight hydrogen peroxide and most preferably from 40 to 70 % by weight.

The epoxidation catalyst can be a homogeneous catalyst or a heterogeneous catalyst. Suitable homogeneous epoxidation catalysts are manganese complexes with polydentate nitrogen ligands, in particular 1,4,7-trimethyl-1,4,7-triazacyclononane ligands, as known from WO 2011/063937. Other suitable homogeneous epoxidation catalysts are heteropolytungstates and heteropolymolybdates, in particular polytungstophosphates, as known from US 5,274,140. Suitable heterogeneous epoxidation catalysts are titanium zeolites containing titanium atoms on silicon lattice positions. Preferably, a titanium silicalite catalyst is used, preferably with an MFI or MEL crystal structure. Most preferably a titanium silicalite 1 catalyst with MFI structure as known from EP 0 100 119 A1, is used. The titanium silicalite catalyst is preferably employed as a shaped catalyst in the form of granules, extrudates or shaped bodies. For the forming process the catalyst may contain 1 to 99% of a binder or carrier material, all binders and carrier materials being suitable that do not react with hydrogen peroxide or with propene oxide under the reaction conditions employed for the epoxidation, silica being preferred as binder. Extrudates with a diameter of 1 to 5 mm are preferably used as fixed bed catalysts.

When the epoxidation catalyst is a titanium silicalite, the propene feed is preferably reacted with hydrogen peroxide in a methanol solvent to provide a liquid reaction mixture comprising methanol. The methanol solvent can be a technical grade methanol, a solvent stream recovered in the work-up of the epoxidation reaction mixture or a mixture of both. The epoxidation reaction is then preferably carried out at a temperature of 30 to 80°C, more preferably at 40 to 60°C. The epoxidation reaction is preferably carried out with addition of ammonia to improve propene oxide selectivity as described in EP 0 230 949 A2. Ammonia is preferably added in an amount of from 100 to 3000 ppm based on the weight of hydrogen peroxide. The epoxidation is preferably carried out in a fixed bed reactor by passing a mixture comprising propene, hydrogen peroxide and methanol over a fixed bed comprising a shaped titanium silicalite catalyst. The fixed bed reactor is preferably equipped with cooling means and cooled with a liquid cooling medium. The temperature profile within this reactor is preferably maintained such that the cooling medium temperature of the cooling means is at least 40°C and the maximum temperature within the catalyst bed is 60°C at the most, preferably 55°C. The epoxidation reaction mixture is preferably passed through the catalyst bed in down flow mode, preferably with a superficial velocity from 1 to 100 m/h, more preferably 5 to 50 m/h, most preferred 5 to 30 m/h. The superficial velocity is defined as the ratio of volume flow rate/cross section of the catalyst bed. Additionally it is preferred to pass the reaction mixture through the catalyst bed with a liquid hourly space velocity (LHSV) from 1 to 20 h⁻¹, preferably 1.3 to 15 h⁻¹. It is particularly preferred to maintain the catalyst bed in a trickle bed state during the epoxidation reaction. Suitable conditions for maintaining the trickle bed state during the epoxidation reaction are disclosed in WO 02/085873 on page 8 line 23 to page 9 line 15. The methanol solvent is preferably used in the epoxidation in a weight ratio of 0.5 to 20 relative to the amount of aqueous hydrogen peroxide solution. The amount of catalyst employed may be varied within wide limits and is preferably chosen so that a hydrogen peroxide consumption of more than 90%, preferably more than 95%, is achieved within 1 minute to 5 hours under the employed epoxidation reaction conditions. Most preferably, the epoxidation reaction is carried out with a catalyst fixed bed maintained in a trickle bed state at a pressure close to the vapor pressure of propene at the reaction temperature, using an excess of propene that provides a reaction mixture comprising two liquid phases, a methanol rich phase and a propene rich liquid phase. Two or more fixed bed reactors may be operated in parallel or in series in order to be able to operate the epoxidation process continuously when regenerating the epoxidation catalyst. Regeneration of the epoxidation catalyst can be carried out by calcination, by treatment with a heated gas, preferably an oxygen containing gas or by a solvent wash, preferably by the periodic regeneration described in WO 2005/000827.

In steps b) and c) of the process of the invention, the liquid reaction mixture resulting from step a) is subjected to a series of at least two sequential pressure reduction steps. When a mixture with two liquid phases, a methanol rich phase and a propene rich liquid phase, is obtained in step a), the methanol rich phase is subjected to the sequential pressure reduction steps. The series of consecutive pressure reduction steps comprises a first pressure reduction step, a last pressure reduction step and optionally one or more intermediate pressure reduction steps. Preferably, the series of sequential pressure reduction steps comprises two or three pressure reduction steps and most preferably, the series comprises two pressure reduction steps. In each pressure reduction step, the pressure is reduced from an initial pressure to a final pressure, which provides a vapor phase, comprising non-reacted propene, and a liquid phase at the final pressure. The final pressure of the last pressure reduction step is less than 0.3 MPa, preferably less than 0.2 MPa. The final pressure of the last pressure reduction step is preferably at least 0.13 MPa. For a series of two pressure reduction steps, the final pressure after the first pressure reduction step is preferably from 0.5 to 0.9 MPa. For each of the pressure reduction steps the vapor phase provided by said step is compressed in a compression step to at least the initial pressure of said pressure reduction step, providing a compressed vapor phase. For each pressure reduction step, except the last pressure reduction step, the vapor phase provided by said step is combined with compressed vapor phase from the subsequent pressure reduction step before performing said compression step. Preferably, all of the compressed vapor phase from the subsequent pressure reduction step is used. For a pressure reduction in two stages with a first and a last pressure reduction step, this means that the vapor phase provided in the last pressure reduction step is compressed, the resulting compressed vapor phase is combined with the vapor phase provided by the first pressure reduction step and the combined vapor phases are compressed to at least the initial pressure of the first pressure reduction step. For a pressure reduction in three stages, the compressed vapor phase from the last pressure reduction step is combined with the vapor phase from the second pressure reduction step, the combined vapor phases are compressed and further combined with the vapor phase from the first pressure reduction step, and all combined vapor phases are then compressed to at least the initial pressure of the first pressure reduction step. Compared to pressure reduction in a single step with recompression of recovered propene, the process of the invention with stepwise pressure reduction and corresponding compressions reduces the energy demand for propene recompression. Compared to processes where non-reacted propene is recovered by distillation at the reaction pressure, the process of the invention reduces heating of the reaction mixture which reduces propene oxide losses due to hydrolysis and solvolysis.

Preferably, for each pressure reduction step except the first pressure reduction step the compressed vapor phase is cooled and liquid condensed by cooling is combined with the liquid phase obtained in the preceding pressure reduction step. This further reduces the energy demand for propene recompression.

The pressure reduction steps are preferably carried out in flash evaporators, i.e. evaporators that do not comprise a heat exchanger. The use of flash evaporators provides adiabatic cooling of the vapor phase resulting from pressure reduction and thereby reduces the need for cooling the compressed vapor phase. The flash evaporators preferably comprise a separator for separating liquid droplets from the vapor phase resulting from pressure reduction. The separator is preferably a demister comprising a high surface material, wetted by the liquid phase, through which the vapor phase is passed. Removing droplets from the vapor phase before compressing it avoids damage to compressors.

The compressed vapor phase from a subsequent pressure reduction step may be introduced directly into the flash evaporator for combining it with the vapor phase resulting from pressure reduction. Preferably, the compressed vapor phase from a subsequent pressure reduction step is cooled and both the cooled vapor and the condensed liquid are introduced into the flash evaporator. In this embodiment, combining vapors for compression and separating liquid condensed from compressed vapor is achieved without extra equipment.

In step d) of the process of the invention, the compressed vapor phase from the first pressure reduction step is condensed in a condensing step to recover non-reacted propene At least part of the recovered non-reacted propene is then recycled to the reaction step. Propene is preferably recycled to the reaction step as a liquid.

The condensing step can be carried out in any type of condenser and is preferably carried out in a distillation column C1 which has a condenser for the overhead vapors. Preferably, the compressed vapor phase from the first pressure reduction step is fed to a lower section of this distillation column and a stream comprising non-reacted propene is withdrawn as the overhead product. The bottoms product of distillation column C1, containing compounds having a boiling point higher than propene, such as propene oxide and optional methanol solvent, is preferably fed to the first pressure reduction step. Carrying out the condensing step in distillation column C1 minimizes recycling of propene oxide product to the reaction step and thereby avoids by-product formation. Passing the bottoms product of distillation column C1 to the first pressure reduction step provides separation of propene, propene oxide and solvent contained in this stream without extra equipment.

When the propene feed to step a) comprises propane, a part or all of the non-reacted propene recovered in the condensing step is preferably fed to a distillation column C2 for separating propane. Distillation column C2 provides a bottoms product enriched in propane and an overhead product that is recycled to the reaction step. Distillation column C2 is preferably designed and operated with a number of theoretical stages and a reflux ratio sufficient to provide a bottoms product containing less than 50 % by weight propene, preferably less than 80 % by weight propene. Preferably, a fraction of the non-reacted propene recovered in the condensing step is fed to distillation column C2 and the remainder is recycled directly to the reaction step, and said fraction and the reflux ratio of distillation column C2 are adjusted to maintain a molar ratio of propane to propene in the propene feed to the reaction step of from 0.08 to 0.12. This allows removal of propane from the process with a small sized distillation column C2 at low energy consumption. Feeding the propene starting material of the process to distillation column C2 at a feed point less than 4 theoretical stages below the column top further reduces the energy consumption for propane removal.

In step e) of the process of the invention, propene oxide is separated from the liquid phase provided in the last pressure reduction step. The separation of propene oxide can be carried out by any method known from the prior art and is preferably carried out using a series of distillations.

When the epoxidation reaction of step a) is carried out in the presence of a methanol solvent, the separation of propene oxide from the liquid phase of the last pressure reduction step in step e) preferably comprises distillation in a pre-separation column to provide an overhead product comprising propene oxide, methanol and residual propene and a bottoms product comprising methanol, water and non-reacted hydrogen peroxide. The pre-separation column is preferably operated to provide an overhead product comprising from 20 to 60 % of the methanol contained in the liquid phase of the last pressure reduction step. The pre-separation column preferably has from 5 to 20 theoretical separation stages in the stripping section and less than 3 theoretical separation stages in a rectifying section and is most preferably operated without reflux and without a rectifying section to minimize the residence time of propene oxide in the pre-separation column. The pre-separation column is preferably operated at a pressure of from 0.16 to 0.3 MPa. Propene oxide and methanol are condensed from the overhead product of the pre-separation column and propene is preferably stripped from the resulting condensate in a propene stripping column which provides a bottom stream comprising propene oxide and methanol which is essentially free of propene. Propene comprising vapor from the propene stripping column and the condenser of the pre-separation column is preferably combined with the vapor phase provided by the last pressure reduction step before this is compressed. This allows recovery of residual propene from the liquid phase of the last pressure reduction step without extra equipment.

Propene oxide is preferably separated from the bottoms stream of the propene stripping column in an extractive distillation using water as the extraction solvent. The extractive distillation is preferably operated with additional feeding of a reactive compound containing an unsubstituted NH₂ group and capable of reacting with acetaldehyde during the extractive distillation, as described in WO 2004/048335. Extractive distillation with a reactive compound provides a high purity propene oxide containing less than 50 ppm of carbonyl compounds.

Methanol can be recovered from the bottoms product of the pre-separation column by distillation. Preferably, the bottoms product of the pre-separation column is subjected to a catalytic hydrogenation with hydrogen to remove non-reacted hydrogen peroxide remaining from step a), as described in WO 03/093255, before methanol is separated by distillation. Such catalytic hydrogenation reduces the amount of carbonyl compounds and acetals in the methanol separated by distillation, which is advantageous when the methanol is recycled to the reaction of step a). The bottoms product of the extractive distillation is preferably combined with the bottoms product of the pre-separation column, preferably before subjecting it to hydrogenation, in order to recover methanol. When hydrazine is used as the reactive compound in the extractive distillation, passing the bottoms product of the extractive distillation to the catalytic hydrogenation will convert non-reacted hydrazine and hydrazones formed from carbonyl compounds to ammonia and amines. The recovered methanol can be recycled as solvent to the reaction of step a). Preferably, the recovered methanol or the bottoms product of the pre-separation column, optionally combined with bottoms product of the extractive distillation and preferably after a catalytic hydrogenation, is treated to remove organic nitrogen compounds as described in WO 2004/048354, more preferably by subjecting it to an acid treatment. Most preferably, the recovered methanol is passed over a cation exchanger in the hydrogen form before it is recycled to the reaction of step a). Removal of organic nitrogen compounds, in particular amines, avoids deactivation of a titanium silicalite catalyst upon recycling of methanol.

In a preferred embodiment of the process of the invention, the liquid reaction mixture from the reaction step is heated in an evaporation step at an evaporation pressure of at least 2.1 MPa and a temperature 5 to 20 °C above the boiling point of propene at this evaporation pressure to provide a propene vapor stream, before subjecting the liquid reaction mixture to the series of pressure reduction steps. The propene vapor stream provided in the evaporation step is preferably passed to the condensing step, preferably to the lower section of a distillation column C1 as described above. The evaporation step is preferably carried out in an evaporator providing a short residence time for the liquid, particularly preferably in a falling film evaporator. The additional evaporation step allows recovery of a part of the non-reacted propene without compressing and thereby saves on energy for compression, without leading to extended heating of the liquid reaction mixture.

The evaporation step is preferably carried out in combination with pressure reduction in flash evaporators. The temperature in the evaporation step can then be set to compensate for part of the adiabatic cooling in the flash evaporators and to adjust the temperature of the liquid phase obtained in the final pressure reduction step. Thereby, formation of ice in the last flash evaporator can be avoided. When the reaction step is operated with a gradual increase in reaction temperature to compensate for deactivation of catalyst, the evaporation step is preferably operated to provide a resulting liquid with an essentially constant temperature in order to maintain a constant load on the compressors of the subsequent pressure reduction steps.

In a preferred embodiment of the process of the invention, when the epoxidation catalyst is a titanium silicalite and the propene feed is reacted with hydrogen peroxide in a methanol solvent, the liquid reaction mixture from the reaction step is stripped with an inert gas in a stripping column before subjecting it to an evaporation step or the series of pressure reduction steps. The stripping removes oxygen contained in the liquid reaction mixture and provides a liquid which is essentially free of oxygen and a strip gas comprising propene, oxygen and inert gas. Suitable inert gases are nitrogen, argon and air depleted in oxygen, with nitrogen being preferred. This stripping allows safe operation of the pressure reduction and compression steps and of condensing step d) without feeding inert gas to these steps, which facilitates complete propene condensation in condensing step d). The strip gas is preferably cooled to condense a stripper condensate and when the condensing step is carried out in a distillation column C1 as described above, the stripper condensate is preferably fed to the lower section of this distillation column. This provides recovery of part of the propene and most of the propene oxide contained in the strip gas without extra equipment. The strip gas, optionally after condensing a stripper condensate, is preferably contacted with a methanol stream in an absorption step to provide a strip gas depleted in propene and a methanol stream enriched in propene. The methanol stream enriched in propene is then passed to the reaction step. The methanol stream used for absorbing propene from the strip gas is preferably a methanol stream separated from the liquid phase provided by the last pressure reduction step. The absorption step is preferably carried out in an absorption column which is preferably operated with counter-current flow. The absorption step is preferably carried out at a pressure which is the same or lower than the pressure in the stripping column and which is in the range of from 1.6 to 2.6 MPa. The temperature in the absorption step is preferably from 20 to 60 °C, more preferably from 30 to 50 °C. Any residual gas from the condensing step is preferably also passed to the absorption step. The absorption step provides efficient recovery of propene from the strip gas without requiring additional compounds for absorbing or additional equipment for recovering propene from an absorbent.

When the epoxidation reaction of step a) is carried out with an excess of propene sufficient to maintain an additional liquid phase rich in propene and a mixture comprising two liquid phases, a methanol rich phase and a propene rich liquid phase, is provided by step a), the methanol rich phase is passed as liquid reaction mixture to the pressure reduction steps of step b) or the optional evaporation step. The condensing step is then preferably carried out in a distillation column C1 and the liquid phase rich in propene provided by step a) is fed to a lower section of said distillation column C1 in addition to the compressed vapor phase from the first pressure reduction step. Propene is thereby recovered from the propene rich liquid phase of step a) without compression.

Figures 1 and 2 show a preferred embodiment of the process of the invention with two pressure reduction steps and a methanol solvent.

A propene feed (1) is continuously reacted with hydrogen peroxide (2) in an epoxidation reactor (3) containing a fixed bed of a titanium silicalite catalyst. The reaction is carried out in a methanol solvent (4) at a pressure of 2.4 MPa. The amount of propene feed is chosen high enough to provide a liquid reaction mixture at the end of the catalyst fixed bed comprising two liquid phases, a propene rich liquid phase (5) and a liquid reaction mixture (6) rich in water and methanol which has a higher density than the propene rich liquid phase (5). The liquid reaction mixture (6) is stripped with nitrogen (7) in a stripping column (8). The stripped reaction mixture (9) is heated in a heat exchanger (10) where a part of the propene dissolved in the stripped reaction mixture (9) is evaporated to form a vapor stream (11). The resulting liquid stream (12) is passed to a first flash evaporator (13) equipped with a demister (14) where the pressure is reduced to 0.6 MPa. The liquid phase (15) obtained in the first flash evaporator is passed to a second flash evaporator (16) equipped with a demister (17) where the pressure is further reduced to 0.13 MPa. The vapor phase (18) formed in the second flash evaporator (16) is compressed with a compressor (19), cooled in condenser (20) and the resulting stream (21) is passed to the first flash evaporator (13), where liquid from stream (21) combines with the liquid formed by depressurizing liquid (12) and vapor from stream (21) combines with the vapor phase formed by depressurizing liquid (12). The vapor phase (22) from first flash evaporator (13) is compressed with a compressor (23) and passed to a lower section of a distillation column C1 (24) operated at 2.1 MPa. A liquid recovered propene stream (25) is obtained from the column condenser (26) as the overhead product. The column bottoms (27) comprising propene oxide, methanol and dissolved propene are passed to the first flash evaporator (13). The strip gas (28) from the stripping column (8) is cooled in a condenser (29) to give a stripper condensate (30) and an off gas stream (31). Vapor stream (11), stripper condensate (30) and the propene rich liquid phase (5) are passed to the lower section of distillation column C1 (24) for recovering the propene contained in these streams.

A part (32) of the recovered propene stream (25) is passed to a distillation column C2 (33) operated at 2.1 MPa where propane is removed with the bottoms product (34) enriched in propane. The remaining part (35) of the recovered propene stream (25) is returned to reactor (3) as part of the propene feed (1). The propene starting material (36) is fed to distillation column C2 (33) near the column top and the overhead product (37) from the column condenser (38) is passed to epoxidation reactor (3) as part of the propene feed (1).

The off gas stream (31) from stripping column (8) and non-condensed gases (39, 40) from column condensers (26, 38) are passed to an absorption column (41), where propene is absorbed in a stream (42) of recovered methanol at a pressure of 2.0 MPa. The resulting methanol stream (43) loaded with propene is returned to epoxidation reactor (3) as part of the methanol solvent (4) and the off gas stream (44) depleted in propene is discharged.

The liquid phase (45) obtained in the second flash evaporator is passed to a pre-separation column (46), where it is separated by distillation to provide an overhead stream (47) comprising propene oxide, methanol and residual propene and a bottoms product (48) comprising methanol, water and non-reacted hydrogen peroxide. A liquid stream (49) comprising propene oxide and methanol is condensed in condenser (50) and propene is stripped from liquid stream (49) in the propene stripping column (51) with overhead stream (52), which is combined with overhead stream (47) of the pre-separation column. Propene is removed with the residual vapor (53) from condenser (50), which is returned to the second flash evaporator (16). The bottoms stream (54) from propene stripping column (51) is passed to propene oxide column (55) where it is subjected to an extractive distillation using an aqueous solution (56) comprising hydrazine as the extraction solvent. Purified propene oxide (57) is obtained as the overhead product.

The bottoms stream (58) from the propene oxide column, comprising water and methanol, is combined with bottoms product (48) from the pre-separation column and passed to a hydrogenation reactor (59) where it is hydrogenated with hydrogen (60) in the presence of a hydrogenation catalyst to remove non-reacted hydrogen peroxide and carbonyl compound by-products. The hydrogenated stream (61) is passed to methanol distillation column (62), where it is separated into a methanol overhead product (63) and a bottoms product (64) comprising water and by-products which is discharged. The methanol overhead product (63) is passed over a cation exchanger (65) in the hydrogen form for removing organic amine traces to provide a recovered methanol stream (66). Part of the recovered methanol stream (66) is passed as stream (42) to absorption column (41) and the remainder, combined with make-up methanol, is passed as stream (67) to epoxidation reactor (3).

### Examples

### Example 1

The energy consumption for recompressing the propene, vaporized during pressure reduction of the epoxidation reaction mixture, to the operating pressure of distillation column C1 (24) was calculated with the simulation program Aspen Plus of Aspen Technology for a process as shown in figures 1 and 2 producing 41.3 t/h propene oxide, operated with a molar ratio of propene to hydrogen peroxide in the epoxidation step of 4.5, a pressure of 2.4 MPa in the reaction step, a pressure reduction to 0.73 MPa in the first pressure reduction step, a pressure reduction to 0.16 MPa in the second pressure reduction step, and a pressure of 2.27 MPa in distillation column C1. The energy consumption for recompressing the propene was calculated as 2.82 MW.

### Example 2

The calculation of example 1 was repeated for a single stage pressure reduction instead a two stage pressure reduction and a corresponding single stage compression. The energy consumption for recompressing the propene was calculated as 5.06 MW.

### List of reference signs:

- 1: Propene feed
- 2: Hydrogen peroxide
- 3: Epoxidation reactor
- 4: Methanol solvent
- 5: Propene rich liquid phase
- 6: Liquid reaction mixture rich in water and methanol
- 7: Nitrogen
- 8: Stripping column
- 9: Stripped reaction mixture
- 10: Heat exchanger
- 11: Vapor stream from heat exchanger (10)
- 12: Liquid stream from heat exchanger (10)
- 13: First flash evaporator
- 14: Demister
- 15: Liquid phase obtained in first flash evaporator
- 16: Second flash evaporator
- 17: Demister
- 18: Vapor phase formed in second flash evaporator
- 19: Compressor
- 20: Condenser
- 21: Stream from condenser (20)
- 22: Vapor phase from first flash evaporator
- 23: Compressor
- 24: Distillation column C1
- 25: Recovered propene stream
- 26: Column condenser of distillation column C1
- 27: Column bottoms from distillation column C1
- 28: Strip gas
- 29: Condenser
- 30: Stripper condensate
- 31: Off gas stream
- 32: Part of recovered propene stream (25)
- 33: Distillation column C2
- 34: Bottoms product from distillation column C2
- 35: Remaining part of recovered propene stream (25)
- 36: Propene starting material
- 37: Overhead product of distillation column C2
- 38: Column condenser of distillation column C2
- 39: Non-condensed gas from column condenser (26)
- 40: Non-condensed gas from column condenser (28)
- 41: Absorption column
- 42: Stream of recovered methanol
- 43: Methanol stream loaded with propene
- 44: Off gas stream depleted in propene
- 45: Liquid phase obtained in second flash evaporator
- 46: Pre-separation column
- 47: Overhead stream from pre-separation column
- 48: Bottoms product from pre-separation column
- 49: Liquid stream comprising propene oxide and methanol
- 50: Condenser
- 51: Propene stripping column
- 52: Overhead stream from propene stripping column
- 53: Residual vapor from condenser (50)
- 54: Bottoms stream from propene stripping column
- 55: Propene oxide column
- 56: Aqueous solution comprising hydrazine
- 57: Purified propene oxide
- 58: Bottoms stream from propene oxide column
- 59: Hydrogenation reactor
- 60: Hydrogen
- 61: Hydrogenated stream
- 62: Methanol distillation column
- 63: Methanol overhead product from methanol distillation column
- 64: Bottoms product from methanol distillation column
- 65: Cation exchanger
- 66: Recovered methanol stream
- 67: Stream to epoxidation reactor (3)

## Claims

1. A process for the epoxidation of propene, comprising
a) continuously reacting a propene feed with hydrogen peroxide in the presence of an epoxidation catalyst in a reaction step at a pressure of at least 1.9 MPa, using propene in excess to hydrogen peroxide, to provide a liquid reaction mixture comprising non-reacted propene;
b) subjecting the liquid reaction mixture to a series of at least two sequential pressure reduction steps, the series comprising a first pressure reduction step and a last pressure reduction step, each pressure reduction step reducing the pressure from an initial pressure to a final pressure and providing a vapor phase, comprising non-reacted propene, and a liquid phase at the final pressure, the final pressure of the last pressure reduction step being less than 0.3 MPa;
c) compressing for each pressure reduction step the vapor phase provided by said step in a compression step to provide a compressed vapor phase having at least the initial pressure of said pressure reduction step, combining for each pressure reduction step, except the last pressure reduction step, the vapor phase provided by said step with compressed vapor phase from the subsequent pressure reduction step before performing said compression step;
d) condensing the compressed vapor phase from the first pressure reduction step in a condensing step to recover non-reacted propene and recycling at least part of the recovered non-reacted propene to the reaction step; and
e) separating propene oxide from the liquid phase provided in the last pressure reduction step.

2. The process of claim 1, wherein said pressure reduction steps are carried out in flash evaporators.

3. The process of claim 2, wherein said flash evaporators comprise demisters removing liquid droplets from said vapor phase.

4. The process of any one of claims 1 to 3, wherein for each pressure reduction step except the first pressure reduction step said compressed vapor phase is cooled and liquid condensed by cooling is combined with the liquid phase obtained in the preceding pressure reduction step.

5. The process of any one of claims 1 to 4, wherein the liquid reaction mixture from the reaction step is heated in an evaporation step at an evaporation pressure of at least 2.1 MPa and a temperature 5 to 20 °C above the boiling point of propene at said evaporation pressure to provide a propene vapor stream before subjecting said liquid reaction mixture to the series of pressure reduction steps and said propene vapor stream is passed to the condensing step.

6. The process of any one of claims 1 to 4, wherein the condensing step is carried out in a distillation column C1, the compressed vapor phase from the first pressure reduction step being fed to a lower section of said distillation column and a stream comprising non-reacted propene being withdrawn as the overhead product.

7. The process of claim 5, wherein the condensing step is carried out in a distillation column C1, the compressed vapor phase from the first pressure reduction step and the propene vapor stream from the evaporation step being fed to a lower section of said distillation column and a stream comprising non-reacted propene being withdrawn as the overhead product.

8. The process of claim 6 or 7, wherein the bottoms product of distillation column C1 is fed to the first pressure reduction step.

9. The process of any one of claims 1 to 8, wherein the propene feed comprises propane, part or all of the non-reacted propene recovered in the condensing step is fed to a distillation column C2 providing a bottoms product enriched in propane and an overhead product that is recycled to the reaction step.

10. The process of any one of claims 1 to 9, wherein the epoxidation catalyst is a titanium silicalite and the propene feed is reacted with hydrogen peroxide in a methanol solvent to provide a liquid reaction mixture comprising methanol.

11. The process of claim 10, wherein the liquid reaction mixture from the reaction step is stripped with an inert gas in a stripping column before subjecting it to an evaporation step or the series of pressure reduction steps, providing a strip gas comprising propene, oxygen and inert gas.

12. The process of claim 11, wherein said strip gas is cooled to condense a stripper condensate, the condensing step is carried out in a distillation column C1, the compressed vapor phase from the first pressure reduction step and the stripper condensate being fed to a lower section of said distillation column and a stream comprising non-reacted propene being withdrawn as the overhead product.

13. The process of claim 11 or 12, wherein a methanol stream is separated from the liquid phase provided by the last pressure reduction step, the strip gas is contacted with said methanol stream in an absorption step providing strip gas depleted in propene and a methanol stream enriched in propene and the methanol stream enriched in propene is passed to the reaction step.

14. The process of claim 13, wherein residual gas from the condensing step is passed to the absorption step.

15. The process of any one of claims 1 to 14, wherein propene is used in the reaction step in an excess sufficient to maintain an additional liquid phase rich in propene, said additional liquid phase is separated from the liquid reaction mixture after the reaction step, the condensing step is carried out in a distillation column C1, the compressed vapor phase from the first pressure reduction step and the additional liquid phase being fed to a lower section of said distillation column C1 and a stream comprising non-reacted propene being withdrawn as the overhead product.

## Patentansprüche

1. Verfahren zur Epoxidierung von Propen, umfassend:
a) kontinuierliches Umsetzen eines Propen-Einsatzstroms mit Wasserstoffperoxid in Gegenwart eines Epoxidationskatalysators in einem Reaktionsschritt bei einem Druck von mindestens 1,9 MPa unter Verwendung von Propen im Überschuss zu Wasserstoffperoxid, unter Erhalt einer flüssigen Reaktionsmischung, die nicht umgesetztes Propen enthält;
b) Anwenden einer Reihe von mindestens zwei aufeinanderfolgenden Druckreduzierungsschritten auf die flüssige Reaktionsmischung, wobei die Reihe einen ersten Druckreduzierungsschritt und einen letzten Druckreduzierungsschritt umfasst, wobei jeder Druckreduzierungsschritt den Druck von einem Anfangsdruck auf einen Enddruck reduziert unter Erhalt einer Dampfphase, die nicht umgesetztes Propen umfasst, und einer flüssigen Phase bei dem Enddruck, wobei der Enddruck des letzten Druckreduzierungsschrittes weniger als 0,3 MPa beträgt;
c) für jeden Druckreduzierungsschritt Komprimieren der durch den Schritt erhaltenen Dampfphase in einem Kompressionsschritt unter Erhalt einer komprimierten Dampfphase mit mindestens dem Anfangsdruck des Druckreduzierungsschritts, wobei für jeden Druckreduzierungsschritt, außer dem letzten Druckreduzierungsschritt, die mit dem Schritt erhaltene Dampfphase mit komprimierter Dampfphase aus dem nachfolgenden Druckreduzierungsschritt kombiniert wird, bevor der Kompressionsschritt durchgeführt wird;
d) Kondensieren der komprimierten Dampfphase aus dem ersten Druckreduzierungsschritt in einem Kondensationsschritt, um nicht umgesetztes Propen wiederzugewinnen und Zurückführen mindestens eines Teils des gewonnenen nicht umgesetzten Propens in den Reaktionsschritt; und
e) Abtrennen von Propenoxid aus der im letzten Druckreduzierungsschritt erhaltenen flüssigen Phase.

2. Verfahren nach Anspruch 1, wobei die Druckreduzierungsschritte in Entspannungsverdampfern durchgeführt werden.

3. Verfahren nach Anspruch 2, wobei die Entspannungsverdampfer Tropfenabscheider umfassen, die Flüssigkeitströpfchen aus der Dampfphase entfernen.

4. Verfahren nach einem der Ansprüche 1 bis 3, wobei für jeden Druckreduzierungsschritt mit Ausnahme des ersten Druckreduzierungsschritts die komprimierte Dampfphase gekühlt wird und durch Kühlung kondensierte Flüssigkeit mit der flüssigen Phase vereinigt wird, die im vorhergehenden Druckreduzierungsschritt erhalten wurde.

5. Verfahren nach einem der Ansprüche 1 bis 4, wobei die flüssige Reaktionsmischung aus dem Reaktionsschritt in einem Verdampfungsschritt bei einem Verdampfungsdruck von mindestens 2,1 MPa und einer Temperatur von 5 bis 20 °C oberhalb des Siedepunkts von Propen bei dem Verdampfungsdruck erhitzt wird unter Erhalt eines Propen-Dampfstroms, bevor die flüssige Reaktionsmischung der Reihe von Druckreduzierungsschritten unterzogen wird, und der Propen-Dampfstrom dem Kondensationsschritt zugeführt wird.

6. Verfahren nach einem der Ansprüche 1 bis 4, wobei der Kondensationsschritt in einer Destillationskolonne C1 durchgeführt wird, wobei die verdichtete Dampfphase aus dem ersten Druckreduzierungsschritt in einen unteren Abschnitt der Destillationskolonne eingespeist wird und ein Strom, der nicht umgesetztes Propen umfasst, als das Kopfprodukt abgezogen wird.

7. Verfahren nach Anspruch 5, wobei der Kondensationsschritt in einer Destillationskolonne C1 durchgeführt wird, wobei die verdichtete Dampfphase aus dem ersten Druckreduzierungsschritt und der Propendampfstrom aus dem Verdampfungsschritt in einen unteren Abschnitt der Destillationskolonne eingespeist werden und ein Strom, der nicht umgesetztes Propen umfasst, als das Kopfprodukt abgezogen wird.

8. Verfahren nach Anspruch 6 oder 7, wobei das Sumpfprodukt der Destillationskolonne C1 dem ersten Druckreduzierungsschritt zugeführt wird.

9. Verfahren nach einem der Ansprüche 1 bis 8, wobei der Propen-Einsatzstroms Propan umfasst, ein Teil oder das gesamte nicht umgesetzte Propen, das in der Kondensationsstufe gewonnen wird, in eine Destillationskolonne C2 eingespeist wird, unter Erhalt eines mit Propan angereicherten Sumpfprodukts und eines Kopfprodukts, das in den Reaktionsschritt zurückgeführt wird.

10. Verfahren nach einem der Ansprüche 1 bis 9, wobei der Epoxidationskatalysator ein Titansilicalit ist und der Propen-Einsatzstrom mit Wasserstoffperoxid in einem Methanol-Lösungsmittel umgesetzt wird, um eine flüssige Reaktionsmischung bereitzustellen, die Methanol enthält.

11. Verfahren nach Anspruch 10, wobei die flüssige Reaktionsmischung aus dem Reaktionsschritt in einer Strippkolonne mit einem Inertgas gestrippt wird, bevor sie einem Verdampfungsschritt oder der Reihe von Druckreduzierungsschritten unterworfen wird, unter Erhalt eines Strippgases, das Propen, Sauerstoff und Inertgas umfasst.

12. Verfahren nach Anspruch 11, wobei das Strippgas gekühlt wird, um ein Strippkondensat zu kondensieren, der Kondensationsschritt in einer Destillationskolonne C1 durchgeführt wird, die verdichtete Dampfphase aus dem ersten Druckreduzierungsschritt und das Strippkondensat in einen unteren Abschnitt der Destillationskolonne eingespeist werden und ein Strom, der nicht umgesetztes Propen umfasst, als Kopfprodukt entnommen wird.

13. Verfahren nach Anspruch 11 oder 12, wobei ein Methanolstrom aus der im letzten Druckreduzierungsschritt erhaltenen flüssigen Phase abgetrennt wird, das Strippgas mit dem Methanolstrom in einem Absorptionsschritt in Kontakt gebracht wird, wobei ein an Propen abgereichertes Strippgas und ein mit Propen angereicherter Methanolstrom erhalten wird, und der mit Propen angereicherte Methanolstrom dem Reaktionsschritt zugeführt wird.

14. Verfahren nach Anspruch 13, wobei das Restgas aus dem Kondensationsschritt in den Absorptionsschritt geleitet wird.

15. Verfahren nach einem der Ansprüche 1 bis 14, wobei Propen in der Reaktionsstufe in einem Überschuss verwendet wird, der ausreicht, um eine zusätzliche Propen-reiche flüssige Phase aufrechtzuerhalten, die zusätzliche Flüssigphase nach der Reaktionsstufe von der flüssigen Reaktionsmischung abgetrennt wird, der Kondensationsschritt in einer Destillationskolonne C1 durchgeführt wird, die verdichtete Dampfphase aus dem ersten Druckreduzierungsschritt und die zusätzliche flüssige Phase in einen unteren Abschnitt der Destillationskolonne C1 eingespeist werden und ein Strom, der nicht umgesetztes Propen enthält, als Kopfprodukt entnommen wird.

## Revendications

1. Procédé d'époxydation de propène, comprenant
a) la réaction continue d'une charge de propène avec du peroxyde d'hydrogène en présence d'un catalyseur d'époxydation dans une étape de réaction à une pression d'au moins 1,9 MPa, en utilisant du propène en excès par rapport au peroxyde d'hydrogène, pour obtenir un mélange de réaction liquide comprenant du propène n'ayant pas réagi ;
b) la soumission du mélange de réaction liquide à une série d'au moins deux étapes de réduction de pression séquentielles, la série comprenant une première étape de réduction de pression et une dernière étape de réduction de pression, chaque étape de réduction de pression diminuant la pression d'une pression initiale à une pression finale et produisant une phase vapeur, comprenant du propène n'ayant pas réagi, et une phase liquide à la pression finale, la pression finale de la dernière étape de réduction de pression étant inférieure à 0,3 MPa ;
c) la compression, pour chaque étape de réduction de pression, de la phase vapeur produite par ladite étape dans une étape de compression pour obtenir une phase vapeur comprimée ayant au moins la pression initiale de ladite étape de réduction de pression, la combinaison pour chaque étape de réduction de pression, à l'exception de la dernière étape de réduction de pression, de la phase vapeur produite par ladite étape avec la phase vapeur comprimée de l'étape de réduction de pression suivante avant d'effectuer ladite étape de compression ;
d) la condensation de la phase vapeur comprimée de la première étape de réduction de pression dans une étape de condensation pour récupérer le propène n'ayant pas réagi et recycler au moins une partie du propène n'ayant pas réagi récupéré dans l'étape de réaction ; et
e) la séparation d'oxyde de propène à partir de la phase liquide produite dans la dernière étape de réduction de pression.

2. Procédé selon la revendication 1, dans lequel lesdites étapes de réduction de pression sont conduites dans des évaporateurs instantanés.

3. Procédé selon la revendication 2, dans lequel lesdits évaporateurs instantanés comprennent des séparateurs de gouttes retirant les gouttelettes de liquide de ladite phase vapeur.

4. Procédé selon l'une quelconque des revendications 1 à 3, dans lequel, pour chaque étape de réduction de pression à l'exception de la première étape de réduction de pression, ladite phase vapeur comprimée est refroidie et le liquide condensé par refroidissement est combiné avec la phase liquide obtenue dans l'étape de réduction de pression précédente.

5. Procédé selon l'une quelconque des revendications 1 à 4, dans lequel le mélange de réaction liquide de l'étape de réaction est chauffé dans une étape d'évaporation à une pression d'évaporation d'au moins 2,1 MPa et une température 5 à 20 °C au-dessus du point d'ébullition du propène à ladite pression d'évaporation pour obtenir un flux de vapeur de propène avant de soumettre ledit mélange de réaction liquide à la série d'étapes de réduction de pression et ledit flux de vapeur de propène est transféré vers l'étape de condensation.

6. Procédé selon l'une quelconque des revendications 1 à 4, dans lequel l'étape de condensation est conduite dans une colonne de distillation C1, la phase vapeur comprimée de la première étape de réduction de pression étant alimentée dans une section inférieure de ladite colonne de distillation et un flux comprenant du propène n'ayant pas réagi étant extrait en tant que produit de tête de distillation.

7. Procédé selon la revendication 5, dans lequel l'étape de condensation est conduite dans une colonne de distillation C1, la phase vapeur comprimée de la première étape de réduction de pression et le flux de vapeur de propène de l'étape d'évaporation étant alimentés dans une section inférieure de ladite colonne de distillation et un flux comprenant du propène n'ayant pas réagi étant extrait en tant que produit de tête de distillation.

8. Procédé selon la revendication 6 ou 7, dans lequel le produit de résidu de colonne de distillation C1 est alimenté dans la première étape de réduction de pression.

9. Procédé selon l'une quelconque des revendications 1 à 8, dans lequel la charge de propène comprend du propane, une partie ou la totalité du propène n'ayant pas réagi récupéré dans l'étape de condensation est alimentée dans une colonne de distillation C2 pour obtenir un produit de résidu enrichi en propane et un produit de tête de distillation qui est recyclé dans l'étape de réaction.

10. Procédé selon l'une quelconque des revendications 1 à 9, dans lequel le catalyseur d'époxydation est un silicalite de titane et la charge de propène réagit avec le peroxyde d'hydrogène dans un solvant méthanolique pour obtenir un mélange de réaction liquide comprenant du méthanol.

11. Procédé selon la revendication 10, dans lequel le mélange de réaction liquide de l'étape de réaction est soumis à un entraînement avec un gaz inerte dans une colonne d'entraînement avant d'être soumis à une étape d'évaporation ou à la série d'étapes de réduction de pression, pour obtenir un gaz d'entraînement comprenant du propène, de l'oxygène et du gaz inerte.

12. Procédé selon la revendication 11, dans lequel ledit gaz d'entraînement est refroidi pour condenser un condensat d'entraînement, l'étape de condensation est conduite dans une colonne de distillation C1, la phase vapeur comprimée de la première étape de réduction de pression et le condensat d'entraînement étant alimenté dans une section inférieure de ladite colonne de distillation et un flux comprenant propène n'ayant pas réagi étant extrait en tant que produit de tête de distillation.

13. Procédé selon la revendication 11 ou 12, dans lequel un flux de méthanol est séparé de la phase liquide produite par la dernière étape de réduction de pression, le gaz d'entraînement est mis en contact avec ledit flux de méthanol dans une étape d'absorption pour obtenir un gaz d'entraînement appauvri en propène et un flux de méthanol enrichi en propène, et le flux de méthanol enrichi en propène est transféré vers l'étape de réaction.

14. Procédé selon la revendication 13, dans lequel le gaz résiduel de l'étape de condensation est transféré vers l'étape d'absorption.

15. Procédé selon l'une quelconque des revendications 1 à 14, dans lequel le propène est utilisé dans l' étape de réaction en un excès suffisant pour maintenir une phase liquide supplémentaire riche en propène, ladite phase liquide supplémentaire est séparée du mélange de réaction liquide après l'étape de réaction, l'étape de condensation est conduite dans une colonne de distillation C1, la phase vapeur comprimée de la première étape de réduction de pression et la phase liquide supplémentaire étant alimentée dans une section inférieure de ladite colonne de distillation C1 et un flux comprenant du propène n'ayant pas réagi étant extrait en tant que produit de tête de distillation.
